# EUROPEAN PATENT SPECIFICATION

(11) **EP 2 773 768 B1**
(45) Date of publication and mention of the grant of the patent: **08.02.2017**
(21) Application number: 12790455.5
(22) Date of filing: 02.11.2012
(51) Int. Cl.: C12Q 1/68

(54) **COMBINATIONS OF MOLECULAR MARKERS IN PROSTATE CANCER PROVIDING A DIAGNOSTIC TOOL WITH IMPROVED SENSITIVITY/SPECIFICITY**
KOMBINATIONEN VON MOLEKULARMARKERN IN PROSTATAKREBS ZUR BEREITSTELLUNG EINES DIAGNOSEWERKZEUGS MIT VERBESSERTER EMPFINDLICHKEIT/SPEZIFITÄT
COMBINAISONS DE MARQUEURS MOLÉCULAIRES DANS LE CANCER DE LA PROSTATE FOURNISSANT UN OUTIL DE DIAGNOSTIC AYANT UNE SENSIBILITÉ/SPÉCIFICITÉ AMÉLIORÉE

(30) Priority: 04.11.2011 WO PCT/EP2011/069448
(43) Date of publication of application: 10.09.2014
(73) Proprietor: MDxHealth Research B.V., 6525 GA Nijmegen (NL)
(72) Inventor: SMIT, Franciscus Petrus, NL-6546 RN Nijmegen (NL); SCHALKEN, Jack A., NL-6524 LH Nijmegen (NL); HESSELS, Daphne, 6546RN Nijmegen (NL)
(74) Representative: van Kooij, Adriaan
(86) International application number: PCT/EP2012/071727
(87) International publication number: WO 2013/064636

(56) References cited:
- WO-A1-2010/037735
- WO-A1-2012/152811
- US-A1- 2009 104 120
- "Affymetrix GeneChip Human Genome U133 Array Set HG-U133A", GEO,, 11 March 2002 (2002-03-11), XP002254749, [retrieved on 2002-03-11]
- PASCAL LAURA E ET AL: "Gene expression relationship between prostate cancer cells of Gleason 3, 4 and normal epithelial cells as revealed by cell type-specific transcriptomes", BMC CANCER, BIOMED CENTRAL, LONDON, GB, vol. 9, no. 1, 18 December 2009 (2009-12-18), page 452, XP021067019, ISSN: 1471-2407, DOI: 10.1186/1471-2407-9-452
- M. CANTILE ET AL: "cAMP induced modifications of HOX D gene expression in prostate cells allow the identification of a chromosomal area involved in vivo with neuroendocrine differentiation of human advanced prostate cancers", JOURNAL OF CELLULAR PHYSIOLOGY, vol. 205, no. 2, 1 January 2005 (2005-01-01), pages 202-210, XP055030612, ISSN: 0021-9541, DOI: 10.1002/jcp.20384

## Description

The present invention relates to methods for in vitro establishing, or diagnosing, high grade or low grade prostate cancer in a sample, preferably from a readily obtainable sample such as an urine, a prostatic fluid or ejaculate sample or a processed, or derived sample thereof, originating from human individual suspected of suffering from prostate cancer using expression level analysis of a combination of two, or three molecular markers for prostate cancer. The present invention further relates to the use in expression level analysis of these combined markers for in vitro establishing high grade or low grade prostate cancer and to a kit of parts providing expression analysis of combinations of the present molecular markers for establishing high grade or low grade prostate cancer.

In the Western male population, prostate cancer has become a major public health problem. In many developed countries it is not only the most commonly diagnosed malignancy, but it is the second leading cause of cancer related deaths in males as well. Because the incidence of prostate cancer increases with age, the number of newly diagnosed cases continues to rise as the life expectancy of the general population increases. In the United States, approximately 218,000 men, and in Europe approximately 382,000 men are newly diagnosed with prostate cancer every year.

Epidemiology studies show that prostate cancer is an indolent disease and that more men die with prostate cancer than from it. However, a significant fraction of the tumours behave aggressively and as a result approximately 32,000 American men and approximately 89,000 European men die from this disease on a yearly basis.

The high mortality rate is a consequence of the fact that there are no curative therapeutic options for metastatic prostate cancer. Androgen ablation is the treatment of choice in men with metastatic disease. Initially, 70 to 80% of the patients with advanced disease show response to the therapy, but with time the majority of the tumours will become androgen independent. As a result most patients will develop progressive disease.

Since there are no effective therapeutic options for advanced prostate cancer, early detection of this tumor is pivotal and can increase the curative success rate. Although the routine use of serum prostate-specific antigen (PSA) testing has undoubtedly increased prostate cancer detection, one of its main drawbacks has been the lack of specificity.

Serum PSA is an excellent marker for prostatic diseases and even modest elevations almost always reflect a disease or perturbation of the prostate gland including benign prostatic hyperplasia (BPH) and prostatitis. Since the advent of frequent PSA testing over 20 years ago, the specificity of PSA for cancer has declined due to the selection of a large number of men who have elevated PSA due to non-cancer mechanisms. This results in a high negative biopsy rate.

Therefore, (non-invasive) molecular tests, that can accurately identify those men who have early stage, clinically localized prostate cancer and who would gain prolonged survival and quality of life from early radical intervention, are urgently needed. Molecular biomarkers identified in tissues can serve as target for new body fluid based molecular tests.

A suitable biomarker preferably fulfils the following criteria:
1) it must be reproducible (intra- en inter-institutional); and
2) it must have an impact on clinical management.

Further, for diagnostic purposes, it is important that the biomarkers are tested in terms of tissue-specificity and discrimination potential between prostate cancer, normal prostate and BPH. Furthermore, it can be expected that (multiple) biomarker-based assays enhance the specificity for cancer detection.

Considering the above, there is an urgent need for molecular prognostic biomarkers for predicting the biological behaviour of prostate cancer and outcome.

For the identification of new candidate markers for prostate cancer, it is necessary to study expression patterns in malignant as well as non-malignant prostate tissues, preferably in relation to other medical data.

Recent developments in the field of molecular techniques have provided new tools that enabled the assessment of both genomic alterations and proteomic alterations in these samples in a comprehensive and rapid manner. These tools have led to the discovery of many new promising biomarkers for prostate cancer. These biomarkers may be instrumental in the development of new tests that have a high specificity in the diagnosis and prognosis of prostate cancer.

For instance, the identification of different chromosomal abnormalities like changes in chromosome number, translocations, deletions, rearrangements and duplications in cells can be studied using fluorescence in situ hybridization (FISH) analysis. Comparative genomic hybridization (CGH) is able to screen the entire genome for large changes in DNA sequence copy number or deletions larger than 10 mega-base pairs. Differential display analysis, serial analysis of gene expression (SAGE), oligonucleotide arrays and cDNA arrays characterize gene expression profiles. These techniques are often used combined with tissue microarray (TMA) for the identification of genes that play an important role in specific biological processes.

Since genetic alterations often lead to mutated or altered proteins, the signalling pathways of a cell may become affected. Eventually, this may lead to a growth-advantage or survival of a cancer cell. Proteomics study the identification of altered proteins in terms of structure, quantity, and post-translational modifications. Disease-related proteins can be directly sequenced and identified in intact whole tissue sections using the matrix-assisted laser desorption-ionization time-of-flight mass spectrometer (MALDI-TOF). Additionally, surface-enhanced laser desorption-ionization (SELDI)-TOF mass spectroscopy (MS) can provide a rapid protein expression profile from tissue cells and body fluids like serum or urine.

In the last years, these molecular tools have led to the identification of hundreds of genes that are believed to be relevant in the development of prostate cancer. Not only have these findings led to more insight in the initiation and progression of prostate cancer, but they have also shown that prostate cancer is a heterogeneous disease.

Several prostate tumours may occur in the prostate of a single patient due to the multifocal nature of the disease. Each of these tumours can show remarkable differences in gene expression and behaviour that are associated with varying prognoses. Therefore, in predicting the outcome of the disease it is more likely that a set of different markers will become clinically important.

Biomarkers can be classified into four different prostate cancer-specific events: genomic alterations, prostate cancer-specific biological processes, epigenetic modifications and genes uniquely expressed in prostate cancer.

One of the strongest epidemiological risk factors for prostate cancer is a positive family history. A study of 44,788 pairs of twins in Denmark, Sweden and Finland has shown that 42% of the prostate cancer cases were attributable to inheritance. Consistently higher risk for the disease has been observed in brothers of affected patients compared to the sons of the same patients. This has led to the hypothesis that there is an X-linked or recessive genetic component involved in the risk for prostate cancer.

Genome-wide scans in affected families implicated at least seven prostate cancer susceptibility loci, HPC1 (1q24), CAPB (1p36), PCAP (1q42), ELAC2 (17p11), HPC20 (20q13), 8p22-23 and HPCX (Xq27-28). Recently, three candidate hereditary prostate cancer genes have been mapped to these loci, HPC1/2'-5'-oligoadenylate dependent ribonuclease L (RNASEL) on chromosome 1q24-25, macrophage scavenger 1 gene (MSR1) located on chromosome 8p22-23, and HPC2/ELAC2 on chromosome 17p11.

It has been estimated that prostate cancer susceptibility genes probably account for only 10% of hereditary prostate cancer cases. Familial prostate cancers are most likely associated with shared environmental factors or more common genetic variants or polymorphisms. Since such variants may occur at high frequencies in the affected population, their impact on prostate cancer risk can be substantial.

Recently, polymorphisms in the genes coding for the androgen-receptor (AR), 5α-reductase type II (SRD5A2), CYP17, CYP3A, vitamin D receptor (VDR), PSA, GST-T1, GST-M1, GST-P1, insulin-like growth factor (IGF-I), and IGF binding protein 3 (IGFBP3) have been studied.

These studies were performed to establish whether these genes can predict the presence of prostate cancer in patients indicated for prostate biopsies due to PSA levels >3 ng/ml. No associations were found between AR, SRD5A2, CYP17, CYP3A4, VDR, GST-M1, GST-P1, and IGFBP3 genotypes and prostate cancer risk. Only GST-T1 and IGF-I polymorphisms were found to be modestly associated with prostate cancer risk.

Unlike the adenomatous polyposis coli (APC) gene in familial colon cancer, none of the mentioned prostate cancer susceptibility genes and loci is by itself responsible for the largest portion of prostate cancers.

Epidemiology studies support the idea that most prostate cancers can be attributed to factors as race, life-style, and diet. The role of gene mutations in known oncogenes and tumour suppressor genes is probably very small in primary prostate cancer. For instance, the frequency of p53 mutations in primary prostate cancer is reported to be low but have been observed in almost 50% of advanced prostate cancers.

Screening men for the presence of cancer-specific gene mutations or polymorphisms is time-consuming and costly. Moreover, it is very ineffective in the detection of primary prostate cancers in the general male population. Therefore, it cannot be applied as a prostate cancer screening test.

Mitochondrial DNA is present in approximately 1,000 to 10,000 copies per cell. Due to these quantities, mitochondrial DNA mutations have been used as target for the analysis of plasma and serum DNA from prostate cancer patients. Recently, mitochondrial DNA mutations were detected in three out of three prostate cancer patients who had the same mitochondrial DNA mutations in their primary tumour. Different urological tumour specimens have to be studied and larger patient groups are needed to define the overall diagnostic sensitivity of this method.

Critical alterations in gene expression can lead to the progression of prostate cancer. Microsatellite alterations, which are polymorphic repetitive DNA sequences, often appear as loss of heterozygosity (LOH) or as microsatellite instability. Defined microsatellite alterations are known in prostate cancer. The clinical utility so far is neglible. Whole genome- and SNP arrays are considered to be powerful discovery tools.

Alterations in DNA, without changing the order of bases in the sequence, often lead to changes in gene expression. These epigenetic modifications include changes such as DNA methylation and histone acetylation/deacetylation. Many gene promoters contain GC-rich regions also known as CpG islands. Abnormal methylation of CpG islands results in decreased transcription of the gene into mRNA.

Recently, it has been suggested that the DNA methylation status may be influenced in early life by environmental exposures, such as nutritional factors or stress, and that this leads to an increased risk for cancer in adults. Changes in DNA methylation patterns have been observed in many human tumours. For the detection of promoter hypermethylation a technique called methylation-specific PCR (MSP) is used. In contrast to microsatellite or LOH analysis, this technique requires a tumour to normal ratio of only 0.1-0.001%. This means that using this technique, hypermethylated alleles from tumour DNA can be detected in the presence of 10⁴ -10⁵ excess amounts of normal alleles.

Therefore, DNA methylation can serve as a useful marker in cancer detection. Recently, there have been many reports on hypermethylated genes in human prostate cancer. Two of these genes are RASSF1A and GSTP1.

Hypermethylation of RASSF1A (ras association domain family protein isoform A) is a common phenomenon in breast cancer, kidney cancer, liver cancer, lung cancer and prostate cancer. The growth of human cancer cells can be reduced when *RASSF1A* is re-expressed. This supports a role for *RASSF1A* as a tumor suppressor gene. Initially no *RASSF1A* hypermethylation was detected in normal prostate tissue. Recently, methylation of the *RASSF1A* gene was observed in both pre-malignant prostatic intra-epithelial neoplasms and benign prostatic epithelia. *RASSF1A* hypermethylation has been observed in 60-74% of prostate tumors and in 18.5% of BPH samples. Furthermore, the methylation frequency is clearly associated with high Gleason score and stage. These findings suggest that *RASSF1A* hypermethylation may distinguish the more aggressive tumors from the indolent ones.

The most described epigenetic alteration in prostate cancer is the hypermethylation of the Glutathione S-transferase P1 (GSTP1) promoter. GSTP1 belongs to the cellular protection system against toxic effects and as such this enzyme is involved in the detoxification of many xenobiotics.

GSTP1 hypermethylation has been reported in approximately 6% of the proliferative inflammatory atrophy (PIA) lesions and in 70% of the PIN lesions. It has been shown that some PIA lesions merge directly with PIN and early carcinoma lesions, although additional studies are necessary to confirm these findings. Hypermethylation of GSTP1 has been detected in more than 90% of prostate tumours, whereas no hypermethylation has been observed in BPH and normal prostate tissues.

Hypermethylation of the GSTP1 gene has been detected in 50% of ejaculates from prostate cancer patients but not in men with BPH. Due to the fact that ejaculates are not always easily obtained from prostate cancer patients, hypermethylation of GSTP1 was determined in urinary sediments obtained from prostate cancer patients after prostate massage. Cancer could be detected in 77% of these sediments.

Moreover, hypermethylation of GSTP1 has been found in urinary sediments after prostate massage in 68% of patients with early confined disease, 78% of patients with locally advanced disease, 29% of patients with PIN and 2% of patients with BPH. These findings resulted in a specificity of 98% and a sensitivity of 73%. The negative predictive value of this test was 80%, which shows that this assay bears great potential to reduce the number of unnecessary biopsies.

Recently, these results were confirmed and a higher frequency of GSTP1 methylation was observed in the urine of men with stage 3 versus stage 2 disease.

Because hypermethylation of GSTP1 has a high specificity for prostate cancer, the presence of GSTP1 hypermethylation in urinary sediments of patients with negative biopsies (33%) and patients with atypia or high-grade PIN (67%) suggests that these patients may have occult prostate cancer.

Recently, a multiplexed assay consisting of 3 methylation markers, *GSTP1*, *RARB*, *APC* and an endogenous control was tested on urine samples from patients with serum PSA concentrations ≥2.5 µg/l. A good correlation of *GSTP1* with the number of prostate cancer-positive cores on biopsy was observed. Furthermore, samples that contained methylation for either *GSTP1* or *RARB* correlated with higher tumor volumes. Methylated genes have the potential to provide a new generation of cancer biomarkers.

Micro-array studies have been very useful and informative to identify genes that are consistently up-regulated or down-regulated in prostate cancer compared with benign prostate tissue. These genes can provide prostate cancer-specific biomarkers and give us more insight into the etiology of the disease.

For the molecular diagnosis of prostate cancer, genes that are highly up-regulated in prostate cancer compared to low or normal expression in normal prostate tissue are of special interest. Such genes could enable the detection of one tumour cell in a huge background of normal cells, and could thus be applied as a diagnostic marker in prostate cancer detection.

Differential gene expression analysis has been successfully used to identify prostate cancer-specific biomarkers by comparing malignant with non-malignant prostate tissues. Recently, a new biostatistical method called cancer outlier profile analysis (COPA) was used to identify genes that are differentially expressed in a subset of prostate cancers. COPA identified strong outlier profiles for v-ets erythroblastosis virus E26 oncogene (*ERG*) and ets variant gene 1 (*ETV1*) in 57% of prostate cancer cases. This was in concordance with the results of a study where prostate cancer-associated *ERG* overexpression was found in 72% of prostate cancer cases. In >90% of the cases that overexpressed either *ERG* or *ETV1* a fusion of the 5' untranslated region of the prostate-specific and androgen-regulated transmembrane-serine protease gene (*TMPRSS2*) with these ETS family members was found. Recently, another fusion between *TMPRSS2* and an ETS family member has been described, the *TMPRSS2-ETV4* fusion, although this fusion is sporadically found in prostate cancers.

Furthermore, a fusion of *TMPRSS2* with *ETV5* was found. Overexpression of *ETV5 in vitro* was shown to induce an invasive transcriptional program. These fusions can explain the aberrant androgen-dependent overexpression of ETS family members in subsets of prostate cancer because *TMPRSS2* is androgen-regulated. The discovery of the *TMPRSS2-ERG* gene fusion and the fact that *ERG* is the most-frequently overexpressed proto-oncogene described in malignant prostate epithelial cells suggests its role in prostate tumorigenesis. Fusions of the 5' untranslated region of the *TMPRSS2* gene with the ETS transcription factors *ERG*, *ETV1* and *ETV4* have been reported in prostate cancer.

Recently, it was shown that non-invasive detection of *TMPRSS2-ERG* fusion transcripts is feasible in urinary sediments obtained after DRE using an RT-PCR-based research assay. Due to the high specificity of the test (93%), the combination of *TMPRSS2-ERG* fusion transcripts with prostate cancer gene 3 (*PCA3*) improved the sensitivity from 62% (*PCA3* alone) to 73% (combined) without compromising the specificity for detecting prostate cancer.

The gene coding for α-methylacyl-CoA racemase (AMACR) on chromosome 5p13 has been found to be consistently up-regulated in prostate cancer. This enzyme plays a critical role in peroxisomal beta oxidation of branched chain fatty acid molecules obtained from dairy and beef. Interestingly, the consumption of dairy and beef has been associated with an increased risk for prostate cancer.

In clinical prostate cancer tissue, a 9-fold over-expression of AMACR mRNA has been found compared to normal prostate tissue. Immunohistochemical (IHC) studies and Western blot analyses have confirmed the up-regulation of AMACR at the protein level. Furthermore, it has been shown that 88% of prostate cancer cases and both untreated metastases and hormone refractory prostate cancers were strongly positive for AMACR. AMACR expression has not been detected in atrophic glands, basal cell hyperplasia and urothelial epithelium or metaplasia. IHC studies also showed that AMACR expression in needle biopsies had a 97% sensitivity and a 100% specificity for prostate cancer detection.

Combined with a staining for p63, a basal cell marker that is absent in prostate cancer, AMACR greatly facilitated the identification of malignant prostate cells. Its high expression and cancer-cell specificity implicate that AMACR may also be a candidate for the development of molecular probes which may facilitate the identification of prostate cancer using non-invasive imaging modalities.

There have been many efforts to develop a body fluid-based assay for AMACR. A small study indicated that *AMACR*-based quantitative real-time PCR analysis on urine samples obtained after prostate massage has the potential to exclude the patients with clinically insignificant disease when *AMACR* mRNA expression is normalized for PSA. Western blot analysis on urine samples obtained after prostate massage had a sensitivity of 100%, a specificity of 58%, a positive predictive value (PPV) of 72%, and a negative predictive value (NPV) of 88% for prostate cancer. These assays using *AMACR* mRNA for the detection of prostate cancer in urine specimens are promising.

Using cDNA micro-array analysis, it has been shown that hepsin, a type II transmembrane serine protease, is one of the most-differentially over-expressed genes in prostate cancer compared to normal prostate tissue and BPH tissue. Using a quantitative real-time PCR analysis it has been shown that hepsin is over-expressed in 90% of prostate cancer tissues. In 59% of the prostate cancers this over-expression was more than 10-fold.

Also there has been a significant correlation between the up-regulation of hepsin and tumour-grade. Further studies will have to determine the tissue-specificity of hepsin and the diagnostic value of this serine protease as a new serum marker. Since hepsin is up-regulated in advanced and more aggressive tumours it suggests a role as a prognostic tissue marker to determine the aggressiveness of a tumour.

Telomerase, a ribonucleoprotein, is involved in the synthesis and repair of telomeres that cap and protect the ends of eukaryotic chromosomes. The human telomeres consist of tandem repeats of the TTAGGG sequence as well as several different binding proteins. During cell division telomeres cannot be fully replicated and will become shorter. Telomerase can lengthen the telomeres and thus prevents the shortening of these structures. Cell division in the absence of telomerase activity will lead to shortening of the telomeres. As a result, the lifespan of the cells becomes limited and this will lead to senescence and cell death.

In tumour cells, including prostate cancer cells, telomeres are significantly shorter than in normal cells. In cancer cells with short telomeres, telomerase activity is required to escape senescence and to allow immortal growth. High telomerase activity has been found in 90% of prostate cancers and was shown to be absent in normal prostate tissue.

In a small study on 36 specimens telomerase activity has been used to detect prostate cancer cells in voided urine or urethral washing after prostate massage. This test had a sensitivity of 58% and a specificity of 100%. The negative predictive value of the test was 55%.

Although it has been a small and preliminary study, the low negative predictive value indicates that telomerase activity measured in urine samples is not very promising in reducing the number of unnecessary biopsies.

The quantification of the catalytic subunit of telomerase, hTERT, showed a median over-expression of hTERT mRNA of 6-fold in prostate cancer tissues compared to normal prostate tissues. A significant relationship was found between hTERT expression and tumour stage, but not with Gleason score. The quantification of hTERT using real-time PCR showed that hTERT could well discriminate prostate cancer tissues from non-malignant prostate tissues. However, hTERT mRNA is expressed in leukocytes, which are regularly present in body fluids such as blood and urine. This may cause false positivity. As such, quantitative measurement of hTERT in body fluids is not very promising as a diagnostic tool for prostate cancer.

Prostate-specific membrane antigen (PSMA) is a transmembrane glycoprotein that is expressed on the surface of prostate epithelial cells. The expression of PSMA appears to be restricted to the prostate. It has been shown that PSMA is upregulated in prostate cancer tissue compared with benign prostate tissues. No overlap in PSMA expression has been found between BPH and prostate cancer, indicating that PSMA is a very promising diagnostic marker.

Recently, it has been shown that high PSMA expression in prostate cancer cases correlated with tumor grade, pathological stage, aneuploidy and biochemical recurrence. Furthermore, increased *PSMA* mRNA expression in primary prostate cancers and metastasis correlated with PSMA protein overexpression. Its clinical utility as a diagnostic or prognostic marker for prostate cancer has been hindered by the lack of a sensitive immunoassay for this protein. However, a combination of ProteinChip® (Ciphergen Biosystems) arrays and SELDI-TOF MS has led to the introduction of a protein biochip immunoassay for the quantification of serum PSMA. It was shown that the average serum PSMA levels for prostate cancer patients were significantly higher compared with those of men with BPH and healthy controls. These findings implicate a role for serum PSMA to distinguish men with BPH from prostate cancer patients. However, further studies are needed to assess its diagnostic value.

A combination of ProteinChip® arrays and SELDI-TOF MS has led to the introduction of a protein biochip immunoassay for the quantification of serum PSMA. It was shown that the average serum PSMA levels for prostate cancer patients were significantly higher compared with those of men with BPH and healthy controls. These findings implicate a role for serum PSMA to distinguish men with BPH from prostate cancer patients. However, further studies are needed to assess its diagnostic value.

RT-PCR studies have shown that PSMA in combination with its splice variant PSM' could be used as a prognostic marker for prostate cancer. In the normal prostate, PSM' expression is higher than PSMA expression. In prostate cancer tissues, the PSMA expression is more dominant. Therefore, the ratio of PSMA to PSM' is highly indicative for disease progression. Designing a quantitative PCR analysis which discriminates between the two PSMA forms could yield another application for PSMA in diagnosis and prognosis of prostate cancer.

Because of its specific expression on prostate epithelial cells and its upregulation in prostate cancer, PSMA has become the target for therapies. The proposed strategies range from targeted toxins and radio nuclides to immunotherapeutic agents. First-generation products have entered clinical testing.

Delta-catenin (p120/CAS), an adhesive junction-associated protein, has been shown to be highly discriminative between BPH and prostate cancer. *In situ* hybridization studies showed the highest expression of δ-catenin transcripts in adenocarcinoma of the prostate and low to no expression in BPH tissue. The average over-expression of δ-catenin in prostate cancer compared to BPH is 15.7 fold.

Both quantitative PCR and in situ hybridization analysis could not find a correlation between δ-catenin expression and Gleason scores.

Increased δ-catenin expression in human prostate cancer results in alterations of cell cycle and survival genes, thereby promoting tumor progression. δ-catenin was detected in cell-free human voided urine prostasomes. The δ-catenin immunoreactivity was significantly increased in the urine of prostate cancer patients. Further studies are needed to assess its potential utility in the diagnosis of prostate cancer.

PCA3, formerly known as DD3, has been identified using differential display analysis. PCA3 was found to be highly over-expressed in prostate tumours compared to normal prostate tissue of the same patient using Northern blot analysis. Moreover, PCA3 was found to be strongly over-expressed in more than 95% of primary prostate cancer specimens and in prostate cancer metastasis. Furthermore, the expression of PCA3 is restricted to prostatic tissue, i.e. no expression has been found in other normal human tissues.

The gene encoding for PCA3 is located on chromosome 9q21.2. The PCA3 mRNA contains a high density of stop-codons. Therefore, it lacks an open reading frame resulting in a non-coding RNA. Recently, a time-resolved quantitative RT-PCR assay (using an internal standard and an external calibration curve) has been developed. The accurate quantification power of this assay showed a median 66-fold up-regulation of PCA3 in prostate cancer tissue compared to normal prostate tissue. Moreover, a median-up-regulation of 11-fold was found in prostate tissues containing less than 10% of prostate cancer cells. This indicated that PCA3 was capable to detect a small number of tumour cells in a huge background of normal cells.

This hypothesis has been tested using the quantitative RT-PCR analysis on voided urine samples. These urine samples were obtained after digital rectal examination (DRE) from a group of 108 men who were indicated for prostate biopsies based on a total serum PSA value of more than 3 ng/ml. This test had 67% sensitivity and 83% specificity using prostatic biopsies as a gold-standard for the presence of a tumour. Furthermore, this test had a negative predictive value of 90%, which indicates that the quantitative determination of PCA3 transcripts in urinary sediments obtained after extensive prostate massage bears great potential in the reduction of the number of invasive TRUS guided biopsies in this population of men.

The tissue-specificity and the high over-expression in prostate tumours indicate that PCA3 is the most prostate cancer-specific gene described so far. Gen-probe Inc. has the exclusive worldwide licence to the PCA3 technology. Multicenter studies using the validated PCA3 assay can provide the first basis for the molecular diagnostics in clinical urological practice.

Modulated expression of cytoplasmic proteins HSP-27 and members of the PKC isoenzyme family have been correlated with prostate cancer progression.

Modulation of expression has clearly identified those cancers that are aggressive - and hence those that may require urgent treatment, irrespective of their morphology. Although not widely employed, antibodies to these proteins are authenticated, are available commercially and are straightforward in their application and interpretation, particularly in conjunction with other reagents as double-stained preparations.

The significance of this group of markers is that they accurately distinguish prostate cancers of aggressive phenotype. Modulated in their expression by invasive cancers, when compared to non-neoplastic prostatic tissues, those malignancies which express either HSP27 or PKCβ at high level invariably exhibit a poor clinical outcome. The mechanism of this association warrants elucidation and validation.

E2F transcription factors, including E2F3 located on chromosome 6p22, directly modulate expression of EZH2. Overexpression of the EZH2 gene has been important in development of human prostate cancer.

EZH2 was identified as a gene overexpressed in hormone-refractory metastatic prostate cancer and showed that patients with clinically localized prostate cancers that express EZH2 have a worse progression than those who do not express the protein.

Using tissue microarrays, expression of high levels of nuclear E2F3 occurs in a high proportion of human prostate cancers but is a rare event in non-neoplastic prostatic epithelium. These data, together with other published information, suggested that the pRB-E2F3-EZH2 control axis may have a crucial role in modulating aggressiveness of individual human prostate cancers.

The prime challenge for molecular diagnostics is the identification of clinically insignificant prostate cancer, i.e. separate the biologically aggressive cancers from the indolent tumours. Furthermore, markers predicting and monitoring the response to treatment are urgently needed.

In current clinical settings over diagnosis and over treatment become more and more manifest, further underlining the need for biomarkers that can aid in the accurate identification of the patients that do not- and do-need treatment.

The use of AMACR immunohistochemistry is now used in the identification of malignant processes in the prostate thus aiding the diagnosis of prostate cancer. Unfortunately, the introduction of molecular markers on tissue as prognostic tool has not been validated for any of the markers discussed.

Experiences over the last two decades have revealed the practical and logistic complexity in translating molecular markers into clinical use. Several prospective efforts, taking into account these issues, are currently ongoing to establish clinical utility of a number of markers. Clearly, tissue biorepositories of well documented specimens, including clinical follow up data, play a pivotal role in the validation process.

Novel body fluid tests based on GSTP1 hypermethylation and the gene PCA3, which is highly over-expressed in prostate cancer, enabled the detection of prostate cancer in non-invasively obtained body fluids such as urine or ejaculates.

WO2010/037735 describes a method for in vitro establishing high grade or low grade prostate cancer comprising determining expression level of one or more genes out of seven genes including HOXC6 and HOXD10, in a tissue sample. The application of new technologies has shown that a large number of genes are up- or down-regulated in prostate cancer.

In the art, there is a continuing need for assays providing establishment, or diagnosis, of low grade, i.e. a Gleason Score of 6 or lower, or high grade, i.e. a Gleason Score of 7 or higher, prostate cancer with maximal sensitivity and specificity.

Sensitivity relates to the assay's ability to identify positive results. In the present context, sensitivity indicates the proportion of individuals suffering from prostate cancer testing positive for low grade or high grade prostate cancer.

Specificity relates to the ability of the test to identify negative results. In the present context, specificity is defined as the proportion of individuals not suffering from low grade or high grade prostate cancer testing negative for it.

It is an object of the present invention, to provide an assay for establishing, or diagnosing, high grade prostate cancer in a sample of a human individual suspected to suffer from prostate cancer thereby aiding in the development of an effective clinical strategy to treat prostate cancer.

Present invention is defined in the appended claims.

Disclosed are an assay and means for performing the assay allowing detecting high and low grade prostate cancer with improved sensitivity/specificity.

Disclosed is a method for in vitro establishing high grade or low grade prostate cancer in a sample originating from a human individual suspected of suffering from prostate cancer comprising:
- determining expression levels of DLX1 and HOXC6; and
- establishing the level of up-regulation of the expression levels of DLX1 and HOXC6 as compared to expression levels of DLX1 and HOXC6 in a sample originating from an individual not suffering from prostate cancer;
   thereby, based on the levels of up-regulation of DLX1 and HOXC6, providing said establishment of high grade or low grade prostate cancer in said sample.

Disclosed is a method for in vitro establishing high grade or low grade prostate cancer in a sample originating from a human individual suspected of suffering from prostate cancer comprising:
- determining the expression levels of DLX1 and HOXD10; and
- establishing the level of up-regulation of the expression level of DLX1 and the level of down-regulation of the expression level of HOXD10 as compared to expression levels of DLX1 and HOXD10 in a sample originating from an individual not suffering from prostate cancer;
   thereby, based on the level of up-regulation of DLX1 and the level of down-regulation of HOXD10, providing said establishment of high grade or low grade prostate cancer in said sample.

Disclosed is a method for in vitro establishing high grade or low grade prostate cancer in a sample originating from a human individual suspected of suffering from prostate cancer comprising:
- determining the expression levels of HOXC6 and HOXD10; and
- establishing the level of up-regulation of the expression level of HOXC6 and the level of down-regulation of the expression level of HOXD10 as compared to expression levels of HOXC6 and HOXD10 in a sample originating from an individual not suffering from prostate cancer;
   thereby, based on the level of up-regulation of HOXC6 and the level of down-regulation of HOXD10, providing said establishment of high grade or low grade prostate cancer in said sample.

Further disclosed is a method for in vitro establishing high grade or low grade prostate cancer in a sample originating from a human individual suspected of suffering from prostate cancer comprising:
- determining the expression levels of DLX1, HOXD10 and HOXC6; and
- establishing the level of up-regulation of the expression levels of DLX1 and HOXC6 and the level of down-regulation of the expression level of HOXD10 as compared to expression levels of DLX1, HOXD10 and HOXC6 in a sample originating from an individual not suffering from prostate cancer;
   thereby, based on the levels of up-regulation of DLX1 and HOXC6 and the level of down-regulation of HOXD10, providing said establishment of high grade or low grade prostate cancer in said sample.

In the present description, reference is made to human genes suitable as biomarkers for prostate cancer by referring to their arbitrarily assigned names. Although the skilled person is readily capable to identify and use the present genes as biomarkers based on these names, the appended figures provide both the cDNA sequence and protein sequences of these genes in the public database. Based on the data provided in the figures, the skilled person, without undue experimentation and using standard molecular biology means, will be capable of determining the expression levels of the indicated biomarkers in a sample thereby providing the present methods.

In the present description, expression level analysis comprises establishing an increased (DLX1, HOXC6) or decreased expression (HOXD10) of a gene as compared to expression of these genes in a similar, equivalent, or corresponding sample originating from a human individual not suffering from prostate tumour cells or prostate tumour tissue, or from an individual not suffering from prostate cancer. In other words, an increased or decreased expression level of a gene according to the present invention is a measure of gene expression relative to a non-disease standard.

For example, establishing an increased expression of DLX1 and HOXC6, as compared to expression of this gene under non-prostate cancer conditions, allows establishing, or diagnosing low grade or high grade prostate cancer thereby providing prognosis and/or prediction of disease survival and an aid to design a clinical treatment protocol.

HOXD10 is a family member of the homeobox (Hox) genes being regulatory genes that direct organogenesis and maintain differentiated tissue function. HOXD10 aids in maintaining a quiescent, differentiated phenotype in endothelial cells by suppressing expression of genes involved in remodeling the extracellular matrix and cell migration.

HOXC6 is also a family member of the homeobox superfamily of genes and the HOX subfamily contain members that are transcription factors involved in controlling and coordinating complex functions during development via spatial and temporal expression patterns. In humans, there are 39 classical HOX genes organized into the clusters A, B, C and D. It has been demonstrated that HOXC6 is crucial to the development and proliferation of epithelial cells in response to hormonal signals.

With respect to HOXC6 expression, at least to transcript variants are known. Within the context of the present invention, HOXC6 expression level determination refers to the combined expression levels of variant 1 and 2.

DLX1 belongs to the family of homeodomain transcription factors which are related to the Drosophila distal-less (Dll) gene. The family has been related to a number of developmental features and appears to be well preserved across species. Dlx genes are implicated in tangential migration of interneurons from the subpallium to the pallium during vertebrate brain development. It has been suggested that Dlx promotes the migration of interneurons by repressing a set of proteins that are normally expressed in terminally differentiated neurons and act to promote the outgrowth of dendrites and axons.

With respect to DLX1 expression, at least to transcript variants are known. Within the context of the present invention, DLX1 expression level determination only refers to determination of the expression level of the variant depicted in the figures.

Determining expression levels comprises determining mRNA expression levels. In other words, determining expression levels comprises determining transcription levels.

Determining expression levels comprises determining protein levels. In other words, determining expression levels comprises determining translation levels.

According to present disclosure establishing low grade prostate cancer comprises establishing prostate cancer with a Gleason Score of 6 or lower and establishing high grade prostate cancer comprises establishing a Gleason Score of 7 or higher.

Low grade prostate cancer (PrCa, Gleason Score equal or less than 6) represents patients with good clinical prognosis. High grade prostate cancer (PrCa, Gleason Score of 7 or more) represents patients with poor clinical prognosis. The group of patients with poor clinical prognosis can be further differentiated in patients having metastases (PrCa Met) and patients who are castration resistant (CRPC) representing a group of patients with aggressive localized disease.

Accordingly, the methods according to the present disclosure preferably relate to further establishing metastasized prostate cancer (PrCa Met) and/or castration resistant prostate cancer (CRPC).

According to a particularly preferred embodiment of the present disclosure, the methods as described above are performed on a sample selected from the group consisting of urine, urine derived, prostatic fluid, prostatic fluid derived, ejaculate and ejaculate derived, an urine, or an urine derived, sample. These samples are the most readily obtainable samples of human bodily derivable samples. However, until the present invention, no reliable diagnostic test has been described using these bodily fluids.

Within the context of the present description, an urine, prostatic fluid or ejaculate derived sample is a sample originating from these bodily fluid, i.e. sample of these fluid further processed, for example, by sedimentation, extraction, precipitation, dilution etc.

Disclosed is the use of a combination of DLX1 and HOXD10 expression level analysis for in vitro establishing low grade or high grade prostate cancer.

Disclosed is the use of a combination of DLX1 and HOXC6 expression level analysis for in vitro establishing low grade or high grade prostate cancer.

Disclosed is the use of a combination of HOXD10 and HOXC6 expression level analysis for in vitro establishing low grade or high grade prostate cancer.

Disclosed is the use of a combination of DLX1, HOXC6 and HOXD10 expression level analysis for in vitro establishing low grade or high grade prostate cancer.

The above aspects of the present disclosure are preferably practised on a sample selected from the group consisting of urine, urine derived, prostatic fluid, prostatic fluid derived, ejaculate and ejaculate derived. on an urine, or an urine derived, sample.

Disclosed is a kit of parts for in vitro establishing high grade or low grade prostate cancer in a sample originating from human individual suspected of suffering from prostate cancer comprising:
- expression level analysis means for determining the expression levels of DLX1 and HOXC6;
- instructions for use.

Disclosed is a kit of parts for in vitro establishing high grade or low grade prostate cancer in a sample originating from human individual suspected of suffering from prostate cancer comprising:
- expression level analysis means for determining the expression levels of DLX1 and HOXD10;
- instructions for use.

Disclosed is a kit of parts for in vitro establishing high grade or low grade prostate cancer in a sample originating from human individual suspected of suffering from prostate cancer comprising:
- expression level analysis means for determining the expression levels of HOXC6 and HOXD10;
- instructions for use.

Disclosed is a kit of parts for in vitro establishing high grade or low grade prostate cancer in a sample originating from human individual suspected of suffering from prostate cancer comprising:
- expression level analysis means for determining the expression levels of DLX1, HOXD10 and HOXC6;
- instructions for use.

In above kits of part according to the present disclosure the expression level analysis means preferably comprise mRNA expression level analysis means, preferably for PCR, rtPCR, NASBA or in situ hybridisation.

In a particular advantageous embodiment, the disclosure provides a method for determining whether a prostate cancer is to be classified as a high grade prostate cancer, the method comprising:
1) determining the expression level of genes DLX1, HOXD10 and HOXC6 in a sample obtained from an individual and expressing each individual expression level as a numeric value;
2) multiplying the three numeric values thus obtained with each other to obtain a multiplied expression value;
3) comparing the multiplied expression value with a predetermined reference value;
   wherein the prostate cancer is classified as a high grade prostate cancer if the multiplied expression value is above the predetermined reference value. This is further herein referred to as the three marker test.

Expression levels of the genes DLX1, HOXD10 and HOXC6 may be obtained in any conventional way known in the art. Preferably they are obtained by quantifying mRNA expression levels.

The expression levels of the three genes are usually expressed as expression levels in relation to a standard level such as a house keeping gene but also absolute levels may be used depending on the method of measurement.

The numeric values obtained are then processed, such as by multiplication with each other meaning the expression level of DLX1 times the inverse of expression level of HOXD10 (invHOXD10), times the expression level of HOXC6. The thus obtained figure is termed multiplied expression value.

The multiplied expression value was found to be a very useful parameter to diagnose high grade prostate cancer. In a population of 234 individuals (58 with high grade prostate cancer, and 176 with either low grade prostate cancer or negative biopsies) it was found that the three marker test outperformed the diagnostic potential of each of the individual marker genes.

The three marker test surprisingly provided a synergistic effect since its diagnostic potential was better than the sum of the parts, i.e. the three genes individually.

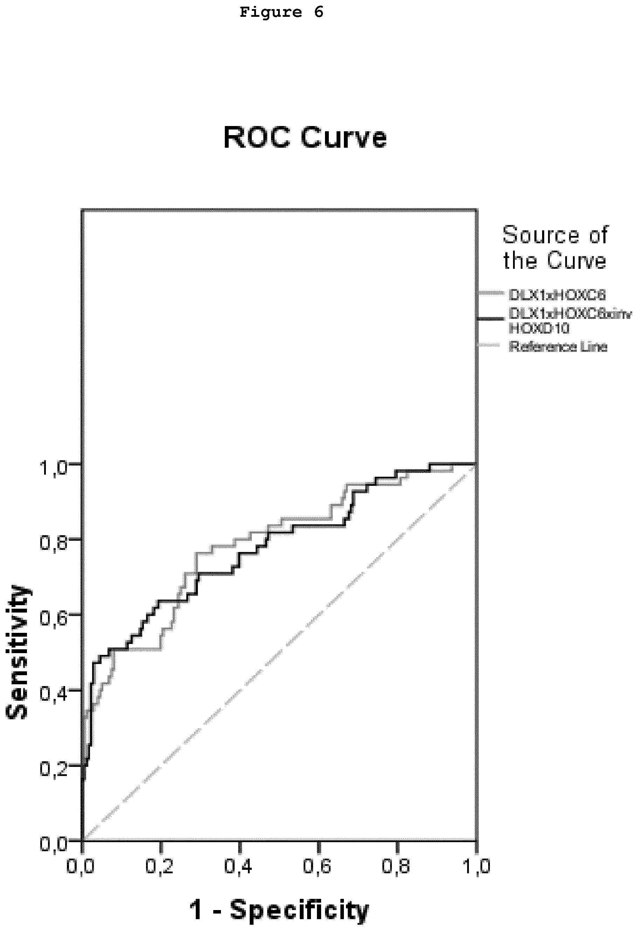

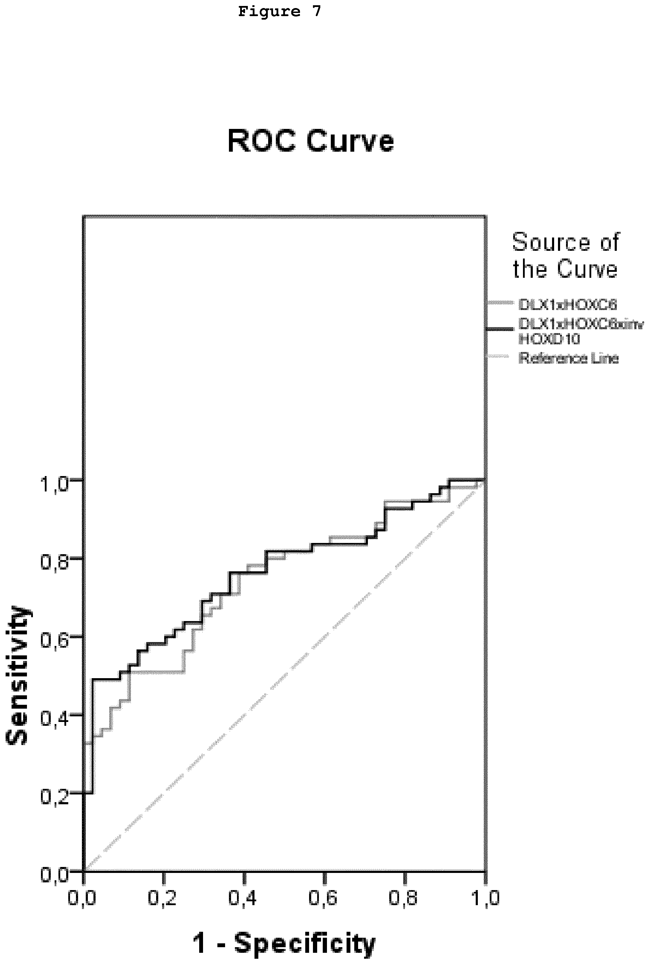

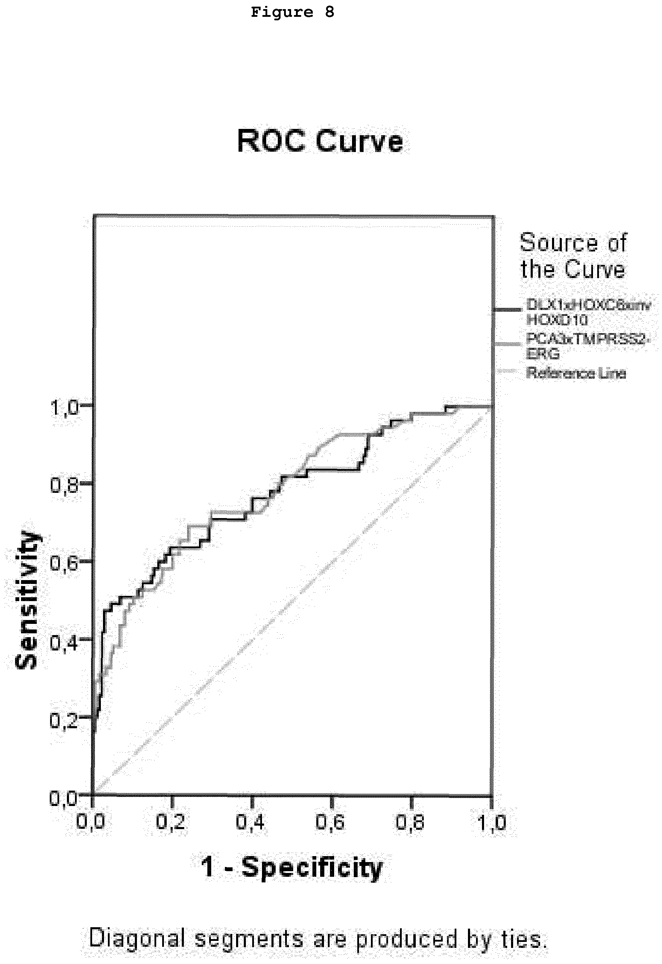

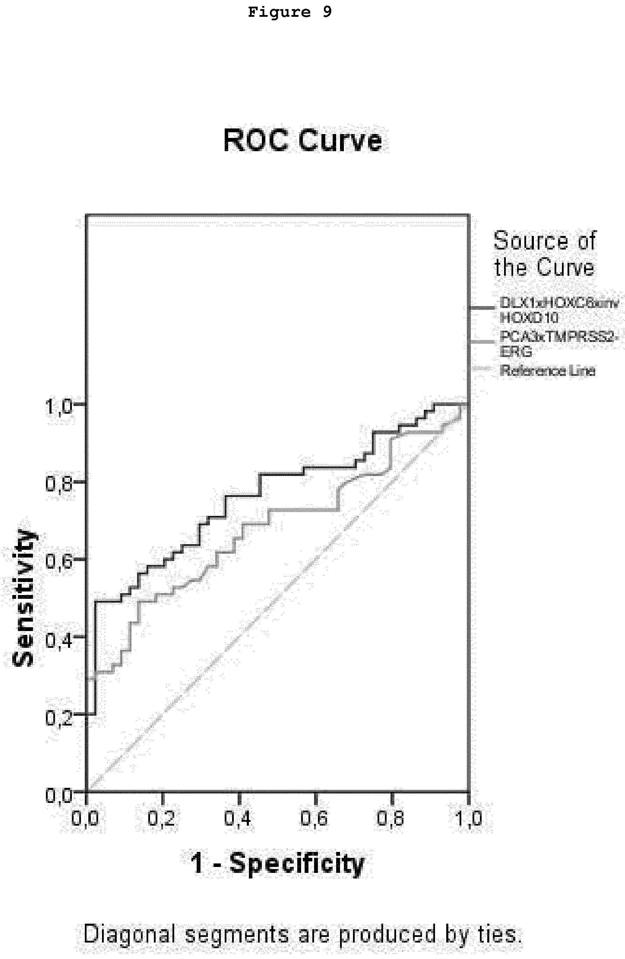

It is evident from figures 6, 7, 8 and 9 that the three marker test outperforms both the two marker test with DLX1 and HOXC6 and a PCA3 and TMPRSS2-ERG two marker reference test, in particular in the range of 75% to 98%, which is a relevant window for diagnosis of high grade prostate cancer.

The predetermined reference value may be experimentally derived using samples from a test population of known high grade and non-high grade prostate cancer. Depending on the desired specificity and sensitivity this value may vary.

One advantageous way of determining the reference value or cut-off value is to determine the mean and standard deviation of multiplied expression values in a number of samples from individuals not suffering from high grade prostate cancer and choosing the mean plus one or two times the standard deviation as the reference value. Any other way of establishing a reference value may provide equally good results.

The method according to the disclosure may also be used as a differentiation assay, i.e. it may be applied in order to distinguish high grade from low grade prostate cancers in a group of individuals already diagnosed with prostate cancer. In this case, the three marker test provides particularly good results in the window of 65% - 98% specificity.

Particularly good results were unexpectedly obtained when the samples were derived from urine, urine sediment, prostatic fluid or ejaculate.

The present invention will be further elucidated in the following detailed example of preferred embodiments of the invention wherein reference is made to figures, wherein:

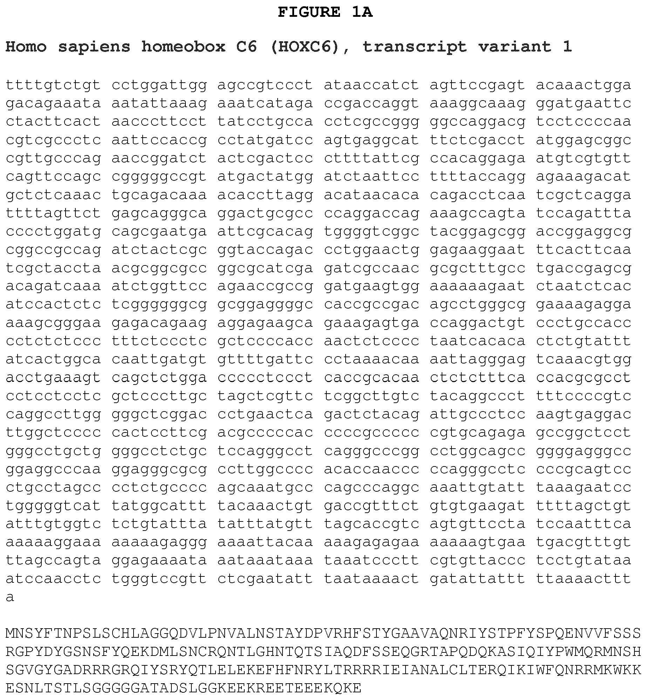

- **Figure 1A**: shows the cDNA and amino acid sequences of the variant 1 of the HOXC6 gene (NM_004503.3, NP_004494.1);

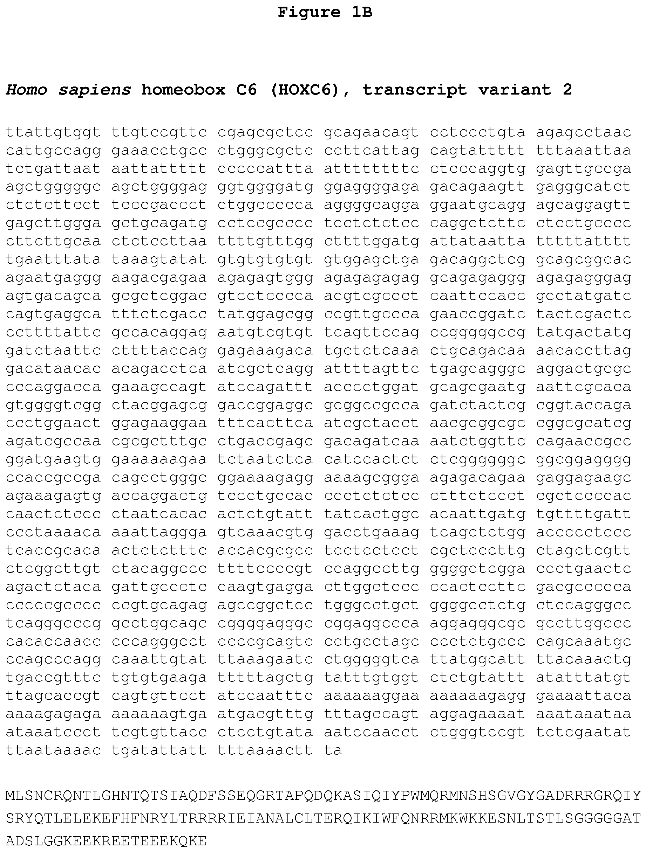

- **Figure 1B**: shows the cDNA and amino acid sequences of the variant 2 of the HOXC6 gene (NM_153693.3, NP_710160.1);

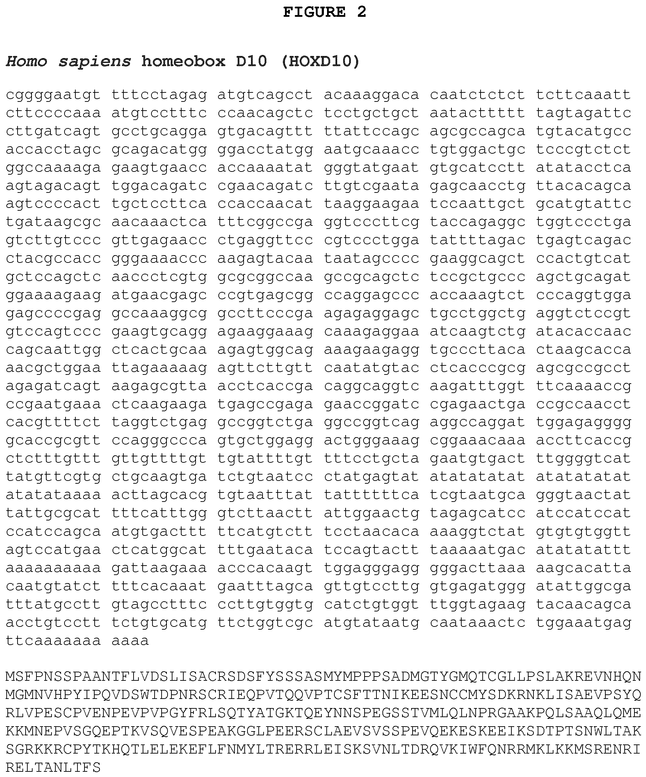

- **Figure 2**: shows the cDNA and amino acid sequences of the HOXD10 gene (NM_002148.3, NP_002139.2);

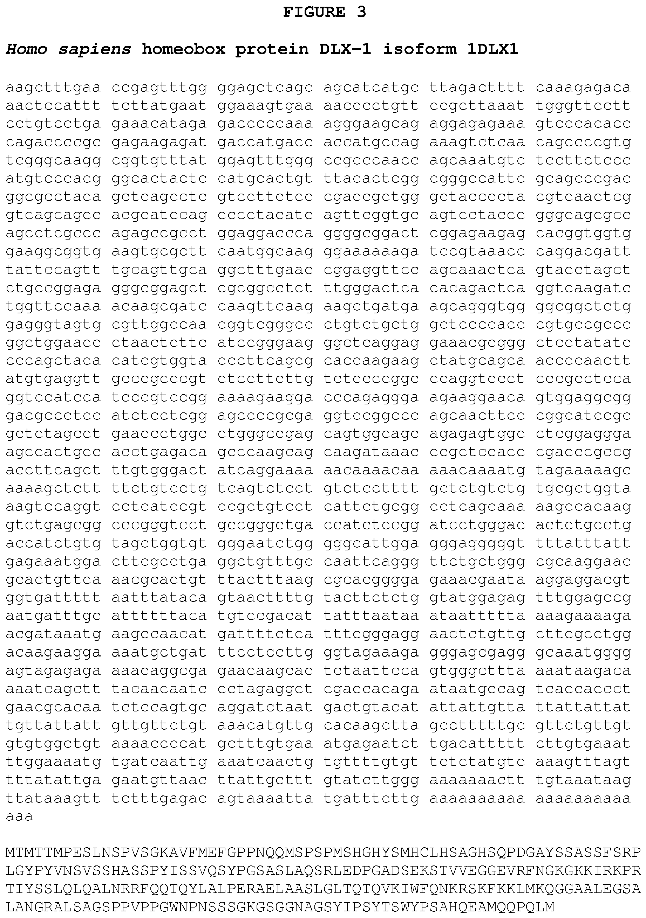

- **Figure 3**: shows the cDNA and amino acid sequences of transcript variant 1 of the DLX1 gene (NM_178120, NP_835221) according to the present invention;
- **Figure 4**: shows discrimination of DLX1 and HOXC6 for high grade prostate tumours (Gleason Score >= 7) as compared to low grade prostate tumours (Gleason Score <= 6) and negative biopsies;
- **Figure 5**: shows discrimination of DLX1 and HOXC6 for high grade prostate tumours (Gleason Score >= 7) as compared to low grade prostate tumours (Gleason Score <= 6);
- **Figure 6**: shows discrimination of a combination of DLX1 and HOXC6 or a combination of DLX1, HOXC6 and HOXD10 for high grade prostate tumours (Gleason Score >= 7) as compared to low grade prostate tumours (Gleason Score <= 6) and negative biopsies.
- **Figure 7**: shows discrimination of a combination of DLX1 and HOXC6 or a combination of DLX1, HOXC6 and HOXD10 for high grade prostate tumours (Gleason Score >= 7) as compared to low grade prostate tumours (Gleason Score <= 6).
- **Figure 8**: shows, compared to a prostate tumour assay, discrimination of a combination of DLX1, HOXC6 and HOXD10 for high grade prostate tumours (Gleason Score >= 7) as compared to low grade prostate tumours (Gleason Score <= 6) and negative biopsies.
- **Figure 9**: shows compared to a prostate tumour assay, discrimination of a combination of DLX1, HOXC6 and HOXD10 for high grade prostate tumours (Gleason Score >= 7) as compared to low grade prostate tumours (Gleason Score <= 6).

### Example

### Materials and Methods

A cohort of 234 consecutive patients that were admitted for prostate biopsies, based on serum PSA levels of more than 3 ng/ml, was tested. After DRE voided urine samples were collected and the mRNA expression levels of DLX1, HOXC6, HOXD10, TMPRSS2-ERG were quantitatively determined in the obtained urinary sediments.

Furthermore, the Progensa PCA3 test was used to determine the PCA3 expression levels in the collected urine specimen. In this cohort, 102 prostate biopsies were positive for prostate cancer. Of all the prostate cancers found, 58 had a Gleason score >=7 en 44 had a Gleason score <=6.

To visualize the efficacy of the present biomarkers to discriminate Gleason score >=7 prostate cancers (n=58) from the Gleason score <=6 and negative biopsies (n=176) in the absence of an arbitrary cut-off value, the data were summarized using a Receiver Operating Characteristic (ROC) curve.

In a ROC curve the true positive rate to detect Gleason score >=7 prostate cancers (Sensitivity) is plotted in function of the false positive rate (i.e. positives in the Gleason score <=6 and negative prostate biopsies population) (100-Specificity) for different cut-off points of a parameter.

Each point on the ROC curve represents a sensitivity/specificity pair corresponding to a particular decision threshold. The area under the ROC curve is a measure of how well a parameter can distinguish between two groups (Gleason score >=7 versus Gleason score <=6 prostate cancers + negative prostate biopsies).

When the variable under study cannot distinguish between the two groups, i.e. in case there is no difference between the two distributions, the area will be equal to 0.5 (the ROC curve will coincide with the diagonal).

A test with perfect discrimination (no overlap in the two distributions) has a ROC curve that passes through the upper left corner (100% sensitivity, 100% specificity). Therefore the closer the ROC curve is to the upper left corner, the higher the overall accuracy of the test.

Furthermore, a ROC-curve was plotted made to visualize the efficacy of the biomarkers to discriminate Gleason score >=7 prostate cancers (n=58) from the Gleason score <=6 prostate cancers (n=44) in the absence of an arbitrary cut-off value.

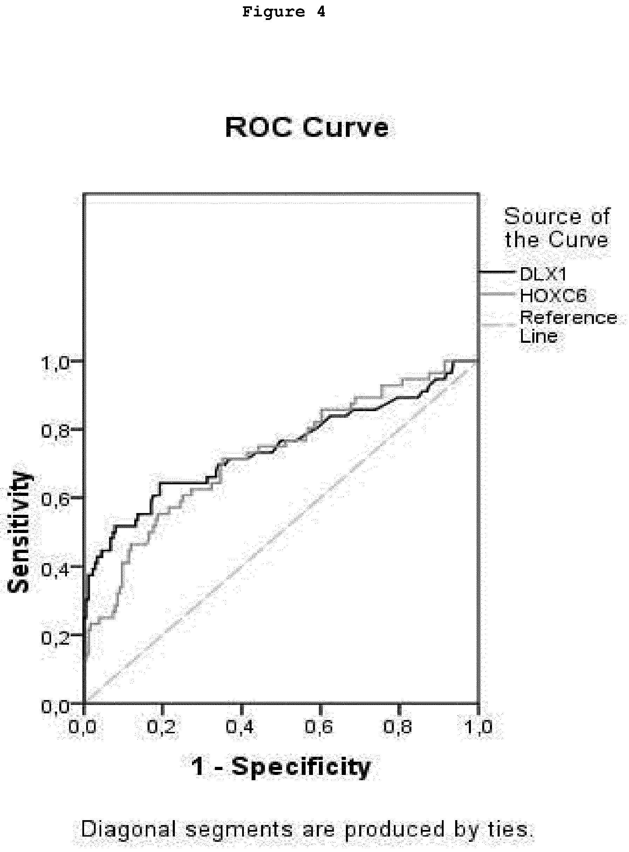

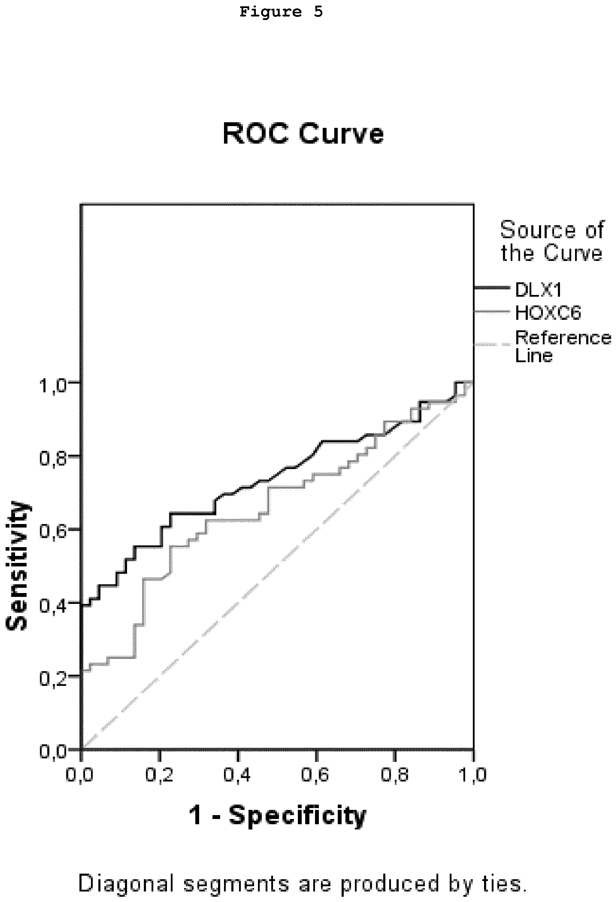

Below, the results obtained and shown in the present **Figures 4** to **9** are discussed

### Results

### Figure 4

In a Receiver under Operation (ROC)-curve, the potential of DLX1 and HOXC6 expression in urinary sediments to discriminate GS >=7 prostate tumours from the rest (GS<=6 and negative biopsies) is visualized. The area under curve (AUC) for DLX-1 is 0.75 (95% CI: 0.66 - 0.83) and for HOXC6 is 0.72 (95% CI: 0.64 - 0.80).

At a specificity >=70%, DLX1 has a significantly higher sensitivity for the detection of GS>=7 tumours from the rest than HOXC6.

### Figure 5

In a Receiver under Operation (ROC)-curve, the potential of DLX1 and HOXC6 expression in urinary sediments to discriminate GS >=7 from GS<=6 prostate tumours is visualized. The area under curve (AUC) for DLX-1 is 0.74 (95% CI: 0.65 - 0.84) and for HOXC6 is 0.66 (95% CI: 0.55 - 0.77). Overall, DLX1 has a significantly higher sensitivity for the detection of GS>=7 tumours from the GS<=6 prostate tumours than HOXC6.

### Figure 6

In a Receiver under Operation (ROC)-curve, the potential of the combination of DLX1 with HOXC6 expression and the combination of DLX1 with HOXC6 and inv(erse)HOXD10 (because of the down regulation correlated with HOXD10 expression; DLX1 and HOXC6 show up regulation) expression in urinary sediments to discriminate GS >=7 prostate tumours from the rest (GS<=6 and negative biopsies) is visualized.

The area under curve (AUC) for the DLX1-HOXC6 combination is 0.78 (95% CI: 0.70 - 0.85) and for DLX1-HOXC6-invHOXD10 is 0.77 (95% CI: 0.69 - 0.85). At a specificity >=80%, the DLX1-HOXC6-invHOXD10 has a higher sensitivity for the detection of GS>=7 tumours from the rest than the DLX1-HOXC6 combination.

### Figure 7

In a Receiver under Operation (ROC)-curve, the potential of the combination of DLX1 with HOXC6 expression and the combination of DLX1 with HOXC6 and invHOXD10 expression in urinary sediments to discriminate GS >=7 prostate tumours from GS<=6 prostate tumours is visualized.

The area under curve (AUC) for the DLX1-HOXC6 combination is 0.74 (95% CI: 0.65 - 0.84) and for DLX1-HOXC6-invHOXD10 is 0.76 (95% CI: 0.67 - 0.85). At a specificity >=75%, the DLX1-HOXC6-invHOXD10 has a higher sensitivity for the detection of GS>=7 tumours from the GS<=6 prostate tumours than the DLX1-HOXC6 combination.

### Figure 8

In a Receiver under Operation (ROC)-curve, the potential of the combination of the combination of DLX1 with HOXC6 and invHOXD10 expression and the combination of PCA3 with TMPRSS2-ERG in urinary sediments to discriminate GS >=7 prostate tumours from the rest (GS<=6 and negative biopsies) is visualized.

The area under curve (AUC) for the DLX1-HOXC6-invHOXD10 combination is 0.78 (95% CI: 0.71 - 0.86) and for the combination PCA3 with TMPRSS2-ERG is 0.78 (95% CI: 0.71 - 0.85). At a specificity >=80%, the DLX1-HOXC6-invHOXD10 has a higher sensitivity for the detection of GS>=7 tumours from the rest than the PCA3_TMPRSS2-ERG combination.

### Figure 9

In a Receiver under Operation (ROC)-curve, the potential of the combination of DLX1 with HOXC6 and invHOXD10 expression and the combination of PCA3 with TMPRSS2-ERG expression in urinary sediments to discriminate GS >=7 prostate tumours from GS<=6 prostate tumours is visualized.

The area under curve (AUC) for the DLX1-HOXC6-invHOXD10 combination is 0.77 (95% CI: 0.68 - 0.86) and for the combination PCA3 with TMPRSS2-ERG is 0.68 (95% CI: 0.57 - 0.78). Overall, the DLX1-HOXC6-invHOXD10 has a higher sensitivity for the detection of GS>=7 tumours from the GS<=6 prostate tumours than the combination PCA3 with TMPRSS2-ERG.

### Discussion

As demonstrated above, the present molecular markers, or biomarkers, for prostate cancer provide, especially in combination, an assay and means for performing the assay allowing detecting high and low grade prostate cancer with improved sensitivity/specificity, especially when compared with presently available biomarkers such as the Progensa PCA3 test.

## Claims

1. Method for *in vitro* establishing high grade prostate cancer in an urine or urine derived sample originating from a human individual suspected of suffering from prostate cancer comprising:
- determining the expression levels of DLX1 and HOXC6; and
- establishing up-regulation of the expression level of DLX1 and HOXC6 as compared to the expression levels of DLX1 and HOXC6 in a sample originating from an individual not suffering from prostate cancer or as compared to a reference value indicative of a non-disease expression level;
thereby, based on up-regulation of the DLX1 and HOXC6 expression levels, providing said establishment of high grade prostate cancer in said sample.

2. Method for *in vitro* establishing high grade prostate cancer in an urine or urine derived sample originating from a human individual suspected of suffering from prostate cancer comprising:
- determining the expression levels of DLX1, HOXD10 and HOXC6; and
- establishing up-regulation of the expression levels of DLX1 and HOXC6 and the level of down-regulation of the expression level of HOXD10 as compared to expression levels of DLX1, HOXD10 and HOXC6 in a sample originating from an individual not suffering from prostate cancer or as compared to a reference value indicative of a non-disease expression level;
thereby, based on up-regulation of DLX1 and HOXC6 and down-regulation of HOXD10, providing said establishment of high grade prostate cancer in said sample.

3. Method according to claim 1 or claim 2, wherein determining said expression levels comprises determining mRNA expression levels.

4. Method according to claim 1 or claim 2, wherein determining said expression levels comprises determining protein levels.

5. Method according to any of the claims 1 to 4, wherein establishing high grade prostate cancer is establishing a Gleason Score of 7 or higher.

6. Method according to claim 5, wherein establishing high grade prostate cancer further comprises establishing metastasized prostate cancer (PrCa Met) and/or castration resistant prostate cancer (CRPC).

## Patentansprüche

1. Verfahren zum In-vitro-Nachweis von Prostatakrebs hohen Grades in einer Urin-oder von Urin abgeleiteten Probe, die von einem menschlichen Individuum stammt, bei dem der Verdacht besteht, dass es an Prostatakrebs leidet, wobei das Verfahren umfasst:
- Bestimmen der Expressionspegel von DLX1 und HOXC6; und
- Bestimmen der Hochregulation des Expressionspegels von DLX1 und HOXC6 verglichen mit den Expressionspegeln von DLX1 und HOXC6 in einer Probe, die von einem Individuum stammt, das nicht an Prostatakrebs leidet, oder verglichen mit einem Referenzwert, der für einen Nicht-Krankheit-Expressionspegel bezeichnend ist;
dadurch, basierend auf der Hochregulation der DLX1- und HOXC6-Expressionspegel, Bereitstellen des Nachweises von Prostatakrebs hohen Grades in der Probe.

2. Verfahren zum In-vitro-Nachweis von Prostatakrebs hohen Grades in einer Urin-oder von Urin abgeleiteten Probe, die von einem menschlichen Individuum stammt, bei dem der Verdacht besteht, dass es an Prostatakrebs leidet, wobei das Verfahren umfasst:
- Bestimmen der Expressionspegel von DLX1, HOXD10 und HOXC6; und
- Bestimmen der Hochregulation der Expressionspegel von DLX1 und HOXC6 und des Pegels der Herunterregulation des Expressionspegels von HOXD10 verglichen mit Expressionspegeln von DLX1, HOXD10 und HOXC6 in einer Probe, die von einem Individuum stammt, das nicht an Prostatakrebs leidet, oder verglichen mit einem Referenzwert, der für einen Nicht-Krankheit-Expressionspegel bezeichnend ist;
dadurch, basierend auf der Hochregulation von DLX1 und HOXC6 und Herunterregulation von HOXD10, Bereitstellen des Nachweises von Prostatakrebs hohen Grades in der Probe.

3. Verfahren gemäß Anspruch 1 oder 2, wobei Bestimmen der Expressionspegel Bestimmen von mRNA-Expressionspegeln umfasst.

4. Verfahren gemäß Anspruch 1 oder 2, wobei Bestimmen der Expressionspegel Bestimmen von Proteinpegeln umfasst.

5. Verfahren gemäß einem der Ansprüche 1 bis 4, wobei Nachweis von Prostatakrebs hohen Grades Nachweis eines Gleason-Score von 7 oder höher bedeutet.

6. Verfahren gemäß Anspruch 5, wobei Nachweis von Prostatakrebs hohen Grades weiter Nachweis von metastasiertem Prostatakrebs (PrCa Met) und/oder kastrationsresistentem Prostatakrebs (CRPC) umfasst.

## Revendications

1. Procédé pour confirmer *in vitro* un cancer de la prostate de haut grade dans un échantillon d'urine ou dérivé de l'urine provenant d'un individu humain suspecté de souffrir d'un cancer de la prostate comprenant :
- la détermination des taux d'expression de DLX1 et de HOXC6 ; et
- la confirmation d'une régulation positive du taux d'expression de DLX1 et de HOXC6 comparativement aux taux d'expression de DLX1 et de HOXC6 dans un échantillon provenant d'un individu ne souffrant pas d'un cancer de la prostate ou comparativement à une valeur de référence indicative d'un taux d'expression non pathologique ;
de cette façon, sur la base de la régulation positive des taux d'expression de DLX1 et de HOXC6, fournir ladite confirmation d'un cancer de la prostate de haut grade dans ledit échantillon.

2. Procédé pour confirmer *in vitro* un cancer de la prostate de haut grade dans un échantillon d'urine ou dérivé de l'urine provenant d'un individu humain suspecté de souffrir d'un cancer de la prostate comprenant :
- la détermination des taux d'expression de DLX1, de HOXD10 et de HOXC6 ; et
- la confirmation d'une régulation positive des taux d'expression de DLX1 et de HOXC6 et du taux de régulation négative du taux d'expression de HOXD10 comparativement aux taux d'expression de DLX1, de HOXD10 et de HOXC6 dans un échantillon provenant d'un individu ne souffrant pas d'un cancer de la prostate ou comparativement à une valeur de référence indicative d'un taux d'expression non pathologique ;
de cette façon, sur la base de la régulation positive des taux d'expression de DLX1 et de HOXC6 et la régulation négative de HOXD10, fournir ladite confirmation d'un cancer de la prostate de haut grade dans ledit échantillon.

3. Procédé selon la revendication 1 ou la revendication 2, dans lequel la détermination desdits taux d'expression comprend la détermination des taux d'expression des ARNm.

4. Procédé selon la revendication 1 ou la revendication 2, dans lequel la détermination desdits taux d'expression comprend la détermination des taux de protéines.

5. Procédé selon l'une quelconque des revendications 1 à 4, dans lequel la confirmation d'un cancer de la prostate de haut grade confirme un score de Gleason de 7 ou supérieur.

6. Procédé selon la revendication 5, dans lequel la confirmation d'un cancer de la prostate de haut grade comprend en outre la confirmation d'un cancer de la prostate métastasé (PrCa Met) et/ou d'un cancer de la prostate résistant à la castration (CRPC)
